# EUROPEAN PATENT SPECIFICATION

(11) **EP 1 244 738 B1**
(45) Date of publication and mention of the grant of the patent: **11.06.2003**
(21) Application number: 00979556.8
(22) Date of filing: 10.11.2000
(51) Int. Cl.: C08K 5/5415, C08L 83/04, A61L 31/06, A61L 29/04

(54) **BIOMIMETIC SILICONE ELASTOMERS**
BIOMIMETISCHE SILOXANELASTOMERE
ELASTOMERES DE SILICONE BIOMIMETIQUES

(30) Priority: 10.11.1999 GB 9926507
(43) Date of publication of application: 02.10.2002
(73) Proprietor: Xiomateria Limited, Belfast BT9 7BL (GB)
(72) Inventor: WOOLFSON, David, Belfast BT9 6TF (GB); MALCOLM, Karl, Belfast BT6 9GA (GB); JONES, David, Newtownabbey BT36 5ZA (GB); GORMAN, Sean, Downpatrick BT30 6NT (GB)
(74) Representative: O'Connell, Maura, Dr.
(86) International application number: EP0011252
(87) International publication number: WO01034695

(56) References cited:
- EP-A- 0 407 867
- EP-A- 0 882 757
- EP-A- 0 916 702
- US-A- 4 247 445
- US-A- 4 838 876

## Description

This invention relates to silicone elastomers produced from polysiloxane vulcanisable compositions and having biomimetic surface properties. The term "biomimetic" as applied to a non-biological surface is intended to embrace properties that are substantially similar to human or animal epithelial tissue *in vivo*. Thus, human or animal epithelial body surfaces are protected by constant renewal through the production and shedding of mucous, which also provides surface lubrication. Biomimetic surface properties, therefore, include resistance to surface microbial growth and infection, resistance to surface deposition of solid material, such as, for example, the deposition of complex inorganic salts, and/or lubricity.

### BACKGROUND OF THE INVENTION

The use of medical devices for temporary or permanent implantation in humans or animals is common, with tens of millions of such devices used annually throughout the world.

By "medical device" is meant any device having a mechanical function, which device is suitable for temporary or permanent implantation in, or for attachment in or on, the human or animal body, the device being selected from, but by no means limited to, urinary tract devices (including urethral stents and urinary catheters), ocular devices (including contact lenses), orthopaedic devices, respiratory devices, cardiovascular devices, dental devices, neurological devices, gastrointestinal devices, audiology devices, surgical devices, including surgical gloves, footcare devices, wound healing devices, condoms. In addition, the term "medical device" also includes devices having drug delivery functions, in addition to the aforementioned mechanical function.

Unfortunately, as the use of such devices increases, so too does the incidence of associated clinical complications such as infection and encrustation. In addition, certain tubular devices such as urinary tract devices present a particular blockage problem, with resultant device failure due to flow obstruction. Urethral catheters may remain in the human or animal body for weeks to years. Long-term catheterisation is associated with an unacceptably high morbidity and mortality, with catheter blockage again representing one of the most common complications in this regard. Catheter care of this type is also time consuming and expensive.

Such medical devices are typically manufactured from one or more biocompatible polymers, collectively referred to as "biomaterials". Silicone elastomer is the most common biomaterial presently used in the production of such devices. However, in common with all presently known biomaterials, silicone elastomer is prone to biofilm formation and, in the case of silicone urinary devices, to encrustation with complex inorganic salts. In addition, silicone elastomer lacks lubricity. Thus, a silicone device is difficult to insert into a body orifice, although this problem can be overcome, to some extent, through the use of a separate lubricating gel. More importantly, for a temporary silicone device, typically in place in the human or animal body for several months, the silicone device may be difficult to remove due to its inherent lack of lubricity. Removal of the silicone device may therefore cause significant epithelial tissue damage.

Device-related infection is a major life-threatening problem with all types of medical devices due to the formation of a microbial biofilm on the biomaterial. When this occurs, antibiotic therapy is unable to eradicate the infection and invasive surgery may be necessary to remove and replace the device.

Silicone elastomers may be prepared by vulcanising a vulcanisable polysiloxane, or a mixture thereof, in the presence of a crosslinking agent, or a mixture thereof, and, if necessary, a condensation catalyst. Suitable vulcanisable polysiloxanes are substantially linear polysiloxanes, containing two, or approximately two, or at least two, terminal silanol (SiOH) groups per molecule and having silicon-bonded alkyl, aryl, alkenyl or halogenated hydrocarbon substituents, which polysiloxanes preferably form silicone elastomers at room temperature, after the addition of crosslinking agent(s) in the presence of a condensation catalyst, for example, stannous octoate.

Certain crosslinking agents having three or four carbon atoms per alkoxy substituent have been disclosed in EP-A-595 531, specifically, tetrapropoxysilane and tetra-n-butoxysilane.

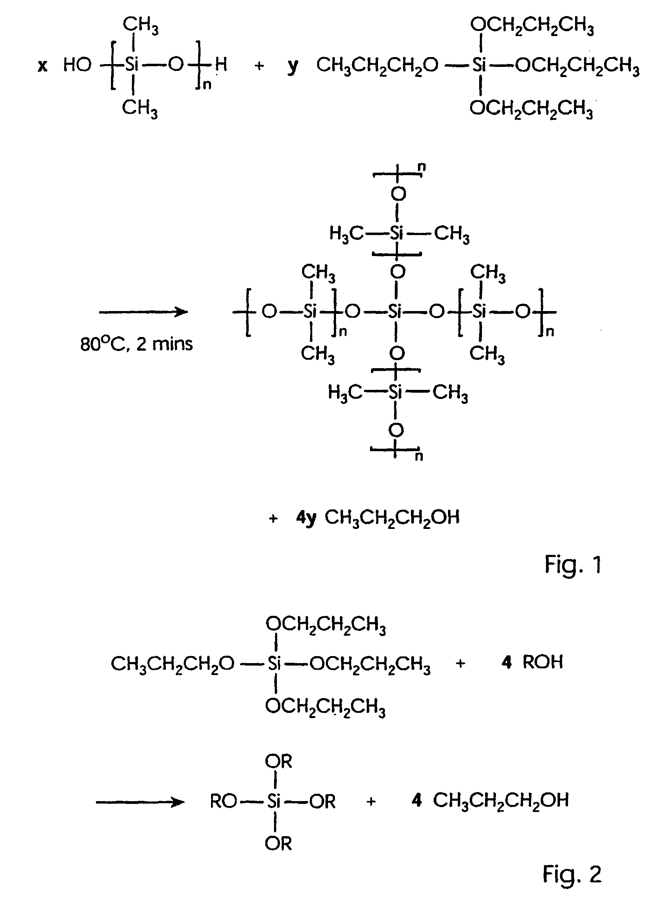

The vulcanising (crosslinking) reaction (Figure 1) proceeds by a condensation reaction whereby, for example, the propoxy groups of the crosslinking agent are replaced by polysiloxane groups, leading to the formation of the crosslinked silicone elastomer, in which the crosslinking agent(s) extend between the substantially linear polysiloxane chains, and a corresponding low molecular weight alcohol - propanol - as a by-product. The propanol migrates to the surface of the elastomer thus formed, the driving force for this migration being the concentration gradient formed by the rapid evaporation of such a low molecular weight alcohol - propanol - from the surface.

When used as biomaterials for the production of medical devices, all presently known silicone elastomers suffer from the disadvantage of an inherent lack of lubricity. In addition, when implanted in a living human or animal body, they are prone to surface attachment of microorganisms and, in certain cases, deposition and attachment of material of biological origin, such as naturally occurring complex inorganic salts.

### OBJECTS OF THE INVENTION

It is a general object of the present invention to provide new and improved biomimetic silicone elastomers produced from vulcanisable polysiloxanes and suitable for use as biomimetic biomaterials for the production of medical devices.

It is a further object of this invention to provide vulcanisable polysiloxanes that may be vulcanised to form silicone elastomers possessing inherent lubricity.

It is a still further object of this invention to provide vulcanisable polysiloxanes that may be vulcanised to form silicone elastomers having resistance to the attachment of microorganisms when implanted or otherwise used in the human or animal body.

It is a more particular object of this invention to provide for an exuding, renewable liquid surface on a silicone elastomer formed from a vulcanisable polysiloxane such that the surface hydrophobicity of the silicone elastomer is altered to yield improved resistance to attachment of microorganisms.

It is a yet more particular object of this invention to provide for an exuding, renewable liquid surface on a silicone elastomer formed from a vulcanisable polysiloxane such that the surface exudate is non-toxic and is readily removed from the surface of the silicone elastomer when present in or on the human or animal body as a medical device, such removal being a natural process due to contact of the silicone elastomer surface with body fluids, or by other natural methods *in vivo*.

### DESCRIPTION OF THE INVENTION

In the present description, the terms "crosslinking", "vulcanisation" and "curing" are used interchangeably to refer to that process by which a viscous, deformable polysiloxane is converted into an elastomer.

### In the drawings,

Figure 1 illustrates the curing reaction scheme for preparing a room temperature vulcanising (RTV) silicone elastomer, using tetrapropoxysilane as the crosslinking agent and stannous octoate as the condensation catalyst;
Figure 2 illustrates the reaction scheme for preparing homoalkoxy, aryloxy or acyloxy silanes of the first aspect of the present invention, using tetrapropoxysilane and an appropriate reagent;
Figure 3 illustrates an apparatus for determining the co-efficient of friction of silicone elastomers of the present invention; and
Figure 4 illustrates the forces required to produce movement of a sled used in the apparatus of Figure 3, at constant speed.

Certain crosslinking agents having three or four carbon atoms per alkoxy substituent have been disclosed in EP-A-595 531, specifically, tetrapropoxysilane and tetra-n-butoxysilane. It has long been thought that crosslinking agents must have relatively low molecular weights, such as those disclosed in EP-A-595 531, due to considerations of steric hindrance which would be expected to prevent the crosslinking reaction from proceeding with higher molecular weight analogues.

Surprisingly, despite a widespread prejudice in the art pointing towards steric hindrance at the reacting sites, it has now been found possible to produce inherently lubricious silicone elastomers, preferably although by no means limited to those of the room temperature vulcanising (RTV) type, using, as at least one crosslinking agent, a silane having homo or hetero alkoxy, homo or hetero aryloxy or homo or hetero acyloxy substituents, or mixtures thereof, so that the resulting by-product of the curing reaction has a higher boiling point component and, therefore, a lower volatility than the alcohol by-products of propanol and butanol which were disclosed in EP-A-595 531.

As will be exemplified hereunder, said by-products of the present invention cannot be merely incorporated directly into the polysiloxane vulcanisable composition, since said by-products inhibit the curing reaction. Although the synthesis of some of such silanes is known in the art [1-5], their use in the curing of polysiloxane vulcanisable compositions is not.

Accordingly, the invention discloses a crosslinking agent, for use in vulcanising a vulcanisable polysiloxane to form a silicone elastomer, the crosslinking agent comprising a silane having four oxygen-bonded substituents, at least one of said oxygen-bonded substituents, which may be the same or different, being selected from functionalised or unfunctionalised, substituted or unsubstituted, straight or branched chain C₅₋₂₅ alkyl, alkenyl or alkynyl; substituted or unsubstituted aryl; or substituted or unsubstituted acyl, in which the moiety bonded to the carboxyl group is a functionalised or unfunctionalised, substituted or unsubstituted, straight or branched chain C₄₋₂₅ alkyl, alkenyl or alkynyl; or substituted or unsubstituted aryl.

In its broadest aspect, therefore, at least one of the oxygen-bonded substituents is as defined above but all remaining oxygen-bonded substituents, which may be the same or different, are not so limited and may be selected from functionalised or unfunctionalised, substituted or unsubstituted, straight or branched chain C₁₋₂₅ alkyl, alkenyl or alkynyl; substituted or unsubstituted aryl; or substituted or unsubstituted acyl, in which the moiety bonded to the carboxyl group is a functionalised or unfunctionalised, substituted or unsubstituted, straight or branched chain C₁₋₂₅ alkyl, alkenyl or alkynyl; or substituted or unsubstituted aryl. Similar comments apply in respect of the first and second aspects of the invention described hereunder.

Preferably, each of said oxygen-bonded substituents, which may be the same or different, is selected from functionalised or unfunctionalised, substituted or unsubstituted, straight or branched chain C₅₋₂₅ alkyl, alkenyl or alkynyl; substituted or unsubstituted aryl; or substituted or unsubstituted acyl, in which the moiety bonded to the carboxyl group is a functionalised or unfunctionalised, substituted or unsubstituted, straight or branched chain C₄₋₂₅ alkyl, alkenyl or alkynyl; or substituted or unsubstituted aryl. More preferably, each of said oxygen-bonded substituents is selected from functionalised or unfunctionalised, substituted or unsubstituted, straight or branched chain C₈₋₂₅ alkyl, alkenyl or alkynyl; substituted or unsubstituted aryl; or substituted or unsubstituted acyl, in which the moiety bonded to the carboxyl group is a functionalised or unfunctionalised, substituted or unsubstituted, straight or branched chain C₈₋₂₅ alkyl, alkenyl or alkynyl; or substituted or unsubstituted aryl.

As used herein, the term "aryl" is intended to embrace substituted or unsubstituted monocyclic or polycyclic aromatic hydrocarbon or heterocyclic radicals. Preferred monocyclic aromatic radicals are selected from phenyl, substituted phenyl radicals such as, but not limited to, tolyl, xylyl, mesityl, cumenyl (isopropylphenyl) and substituted phenylene derivatives such as, but not limited to, benzyl, benzhydryl, cinnamyl, phenethyl, styryl and trityl. Preferred polycyclic aromatic radicals include, but are not limited to, substituted or unsubstituted naphthalene and anthracene radicals, of which napthyl is most preferred.

As used herein, the term "acyl" is intended to embrace all radicals of the structural formula 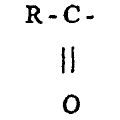 in which R, being the moiety bonded to the carboxyl group, is a functionalised or unfunctionalised, substituted or unsubstituted, saturated straight or branched chain C₁₋₂₅ alkyl; a functionalised or unfunctionalised, substituted or unsubstituted, unsaturated, straight or branched chain C₂₋₂₅ moiety (including alkenyl and alkynyl); or is a substituted or unsubstituted aryl.

As used herein, the term "functionalised" indicates the presence of reactive chemical moieties such as, but not limited to, halide, hydroxyl, carboxyl, carbonyl, anhydride, cyano, ester, ether, sulphide, thiocyanate, thioether, alkene, alkyne and various conjugated groups and the term "unfunctionalised" indicates their absence.

The invention provides, in a first aspect, a polysiloxane vulcanisable composition comprising a vulcanisable polysiloxane or a mixture thereof; a crosslinking agent, or a mixture thereof, the crosslinking agent or the mixture thereof including a silane having four oxygen-bonded substituents, at least one of said oxygen-bonded substituents, which may be the same or different, being selected from functionalised or unfunctionalised, substituted or unsubstituted, straight or branched chain C₅-C₂₅ alkyl; functionalised or unfunctionalised, substituted or unsubstituted C₅₋₂₅ alkenyl or alkynyl; substituted or unsubstituted aryl; or substituted or unsubstituted acyl, in which the moiety bonded to the carboxyl group is a functionalised or unfunctionalised, substituted or unsubstituted, straight or branched chain C₄₋₂₅ alkyl; functionalised or unfunctionalised, substituted or unsubstituted, straight or branched chain C₄₋₂₅ alkenyl or alkynyl or substituted or unsubstituted aryl; and, if necessary, a condensation catalyst

The invention, in a second aspect, provides a silicone elastomer formed by vulcanising a vulcanisable polysiloxane, or a mixture thereof, in the presence of a crosslinking agent, or a mixture thereof, the crosslinking agent or the mixture thereof including a silane having four oxygen-bonded substituents, at least one of said oxygen-bonded substituents, which may be the same or different, being selected from functionalised or unfunctionalised, substituted or unsubstituted, straight or branched chain C₅-C₂₅ alkyl; functionalised or unfunctionalised, substituted or unsubstituted, straight or branched chain C₅₋₂₅ alkenyl or alkynyl; substituted or unsubstituted aryl; or substituted or unsubstituted acyl in which the moiety bonded to the carboxyl group is a functionalised or unfunctionalised, substituted or unsubstituted, straight or branched chain C₄₋₂₅ alkyl; functionalised or unfimctionalised, substituted or unsubstituted, straight or branched chain C₄₋₂₅ alkenyl or alkynyl or substituted or unsubstituted aryl and, if necessary, a condensation catalyst.

In another aspect, the invention provides a medical device comprising a silicone elastomer according to the second aspect of the invention.

In another aspect of the invention, there is provided use of a silicone elastomer according to the second aspect of the invention in a medical device for permanent or temporary implantation in, or for attachment in or on, the human or animal body.

In a still further aspect of the invention, there is provided use of a silicone elastomer according to the second aspect of the invention for the manufacture of a medical device for temporary or permanent implantation in, or for attachment in or on, the human or animal body, in which the silicone elastomers act as biomimetic biomaterials.

It has surprisingly been found that the oily alcoholic by-product of the crosslinking reaction (see Figure 1) is slowly and continually exuded onto the surface of the formed silicone elastomer, producing a lubricious, mobile oily coat of low volatility. This mobile oily coat has the following advantages when the resulting elastomer is used as a biomaterial in a medical device:-
a) Production of a pharmaceutically acceptable, self-lubricating silicone elastomer which, when used in the form of an insertable medical device, offers ease of insertion and removal, compared to medical devices fabricated from conventional silicone elastomers.
b) Slow, substantially constant pharmaceutically acceptable alcoholic by-product exudate is released over prolonged periods from the matrix of the silicone elastomer.
c) The hydrophobicity of the silicone elastomer surface is altered by the formation of a mobile coating that is less hydrophobic due to the presence of alcoholic (hydroxyl) groups, thus rendering the surface less attractive to the attachment of hydrophobic adherents, including certain microorganisms.
d) The pharmaceutically acceptable mobile fluid coating protects the silicone elastomer surface from organic or inorganic adherents, and is then shed from the surface, when placed in, or on, the human or animal body, by the action of body fluids and/or abrasion, such surface coating being constantly replaced by new material from within the body of the elastomer. This process is biomimetic, in that it mimics the constant shedding of the mucin coating on epithelial tissue that serves to protect such tissues from potentially harmful adherents.

### Vulcanisable Polysiloxane

The vulcanisable polysiloxane may be any of those suitable for use in polysiloxane vulcanisable compositions. Preferably, such polysiloxanes are substantially linear diorganopolysiloxanes containing two, or about two, or at least two, terminal silanols (silicon-bonded hydroxyl groups) per molecule. The organo groups present in such diorganopolysiloxanes are selected from C₁₋₁₈ linear, branched or cyclic alkyl radicals such as methyl, ethyl, propyl, isopropyl, butyl, isobutyl, secbutyl, pentyl, hexyl, ethylhexyl, heptyl, octyl, nonyl, decyl, undecyl, dodecyl, tridecyl, tetradecyl, pentadecyl, hexadecyl, heptadecyl or octadecyl radicals; C₂₋₁₀ linear, branched or cyclic alkenyl radicals such as vinyl, allyl, isopropenyl, butenyl, pentenyl, hexenyl, heptenyl, octenyl, nonenyl, decenyl, or cyclohexenyl; aryl monocyclic and polycyclic radicals such as phenyl or naphthyl; and halogenated hydrocarbon (alkyl, alkenyl or aryl) radicals such as chlorophenyl, bromophenyl or trihalopropyl radicals. Most preferred are dimethylpolysiloxanes.

A particularly preferred vulcanisable polysiloxane comprises a mixture of low and high molecular weight poly(dimethylsiloxane)s having terminal silicon-bonded hydroxyl groups. One such commercially available material is Nu-Sil Med-6382 (Trade Mark of Nusil Technology, Inc.). Such preferred polysiloxane materials may contain known reinforcing fillers such as diatomaceous earth and, optionally, known processing aids such as viscosity adjusting agents, for example, silicone or mineral oils. It will, of course, be appreciated that the use of a viscosity reducing agent may be avoided by using different chain lengths of the polydimethyl siloxanes to alter viscosity, if necessary. Furthermore, if a viscosity reducing agent is required, it is preferred that a mineral oil such as, for example, liquid paraffin is used.

### Crosslinking Agent

The proportion of the, or each, crosslinking agent present in the vulcanisable composition, may be varied according to the desired properties of the novel silicone elastomer to be produced. Preferably, the proportion of crosslinking agent, or agents, employed lies in the range of 1 to 25 parts, preferably 2 to 25 parts, more preferably 2 to 15 parts, most preferably 2 to 12 parts, by weight, per 100 parts by weight of the vulcanisable polysiloxane.

Preferably, at least one of the crosslinking agents for use in the present invention is a tetra-alkoxysilane. More preferably, each such alkoxy group has an identical number of carbon atoms, yielding a homo tetra-alkoxysilane, subject then to the proviso that each such alkoxy group has more than four carbon atoms. More preferably, the homo tetra-alkoxysilane should contain at least six, preferably at least eight, carbon atoms per alkoxy group.

It will, however, be realised by those skilled in the art that hetero alkoxysilanes, in which one or more of the alkoxy groups are not identical, also fall within the scope of the invention when used as a crosslinking agent to produce the novel biomimetic silicone elastomers described herein. At least one such alkoxy group must possess and, preferably all such alkoxy groups possesses, greater than four carbon atoms. Most preferably all such alkoxy groups possess at least six, even more preferably at least eight, carbon atoms.

Suitable alkoxy groups include, but are not limited to, C₁₋₂₅ alkyl radicals such as methyl, ethyl, propyl, isopropyl, butyl, isobutyl, sec-butyl, pentyl, isopentyl, hexyl, methylpentyl, isohexyl, heptyl, isoheptyl, octyl, isooctyl, nonyl, isononyl, decyl, isodecyl, undecyl, dodecyl, tridecyl, tetradecyl, pentadecyl, hexadecyl, heptadecyl, octodecyl, nonadecyl, icosyl, henicosyl, docosyl, tricosyl, tetracosyl and pentacosyl, all of which may be further substituted or functionalised . Further suitable alkoxy groups include, but are not limited to, C₂₋₂₅ alkenyl or alkynyl radicals such as vinyl, ethynyl, allyl, isopropenyl, propynyl, butenyl, butynyl, pentenyl, pentynyl, hexenyl, hexynyl, heptenyl, heptynyl, octenyl, octynyl, nonenyl, nonynyl, decenyl, decynyl, undecenyl, undecynyl, dodecenyl, dodecynyl, tridecenyl, tridecynyl, tetradecenyl, tetradecynyl, pentadecenyl, pentadecynyl, hexadecenyl, hexadecynyl, heptadecenyl, heptadecynyl, octadecenyl (oleic or elaidic) octadecynyl, nonadecenyl, nonadecynyl, icosenyl, icosynyl, henicosenyl, henicosynyl, docosenyl, docosynyl, tricosenyl, tricosynyl, tetracosenyl, tetracosynyl, pentacosenyl, and pentacosynyl, all of which may be further substituted or functionalised. In each case, it is necessary that at least one oxygen-bonded substituent must contain greater than four carbon atoms.

It will also be appreciated that silanes containing mixtures of alkoxy, aryloxy and /or acyloxy groups, are also envisaged as suitable crosslinking agents. At least one such group must possess, and preferably all such groups possesses, greater than four carbon atoms, more preferably at least six, and most preferably at least eight, carbon atoms.

As used herein, the term "aryl" is intended to embrace substituted or unsubstituted monocyclic or polycyclic aromatic hydrocarbon or heterocyclic radicals.
Preferred monocyclic aromatic radicals are selected from phenyl, substituted phenyl radicals such as, but not limited to, tolyl, xylyl, mesityl, cumenyl (isopropylphenyl) and substituted phenylene derivatives such as, but not limited to, benzyl, benzhydryl, cinnamyl, phenethyl, styryl and trityl. Preferred polycyclic aromatic radicals include, but are not limited to, substituted or unsubstituted naphthalene and anthracene radicals, of which napthyl is most preferred.

Aryl radicals for use in the present invention may be mono or multi-substituted with a range of chemical moieties, including, but not limited to halogen (fluoride, chloride, bromide or iodide, of which chloride is preferred), straight or branched, saturated or unsaturated, functionalised or unfunctionalised alkyl, and any of the reactive chemical moieties previously disclosed (hydroxyl, carboxyl, carbonyl, anhydride, cyano, ester, ether, sulphide, sulphone, thiocyanate, thioether, alkene, alkyne, various conjugated groups and poly(ethyleneoxide) chains).

Acyl radicals may be functionalised or unfunctionalised, substituted or unsubstituted, saturated or unsaturated, straight or branched chain radicals of the formula 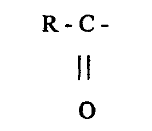 in which R is a functionalised or unfunctionalised, saturated, straight or branched chain C₁₋₂₅ alkyl; a functionalised or unfunctionalised, unsaturated, straight or branched chain C₂₋₂₅ alkenyl or alkynyl; or is a substituted or unsubstituted aryl. Examples of acyl radicals for use in the present invention include, but are not limited to, unsubstituted benzoyl, pentoyl, oleoyl, decoyl, and furanoyl.

Preferably, the acyloxysilane used as the, or one of the, crosslinking agents in the present invention is a tetra-acyloxy silane. More preferably, each acyloxy group has an identical number of carbon atoms, yielding a homo tetra-acyloxysilane. Most preferably, each acyloxy group has greater than four carbon atoms, more preferably greater than six, even more preferably greater than eight, carbon atoms. It will, however, be realised by those skilled in the art that hetero acyloxysilanes, in which one or more of the acyloxy groups are not identical are also envisaged as suitable crosslinking agents.

It will be appreciated that silanes containing mixtures of alkoxy and acyloxy groups; silanes containing mixtures of alkoxy, aryloxy and acyloxy groups; and silanes containing mixtures of aryloxy and acyloxy groups are also envisaged as suitable cross-linking agents.

The benefits of the present invention can be accomplished by either including a crosslinking agent, or a mixture thereof, as disclosed in the present invention with a known crosslinking agent such as tetrapropoxysilane or tetra n-butoxysilane or, alternatively, by replacing the known crosslinking agent with a crosslinking agent, or a mixture thereof, as disclosed in the present invention (described herein).

Thus, the use, in admixture, of one or more silane crosslinking agents with homo alkoxy, hetero alkoxy, homo or hetero aryloxy and/or homo or hetero aryloxy oxygen-bonded substituents, as described herein, in combination with the previously disclosed crosslinking agents (tetrapropoxysilane and tetra-n-butoxysilane), is also disclosed herein. Such combinations should preferably contain (by weight) between 10 and 99%, more preferably 40-99%, and still more preferably 80% to 95%, by weight of the crosslinking agent(s) as disclosed in the present invention, with reference to the total concentration of crosslinking agent(s). More preferably, the crosslinking agent(s) as disclosed in the present invention used in such combinations should be a homo tetra-alkoxysilane in which each alkoxy group has at least four carbon atoms, more preferably, at least six carbon atoms, most preferably at least eight carbon atoms.

The alkoxy, aryloxy or carboxy silanes, to be used as crosslinking agent(s), may be synthesised by any means known in the art.

### Condensation Catalyst

The condensation catalyst, if employed, may be one or more of organic acids, organic bases and metal salts of carboxylic acids. The choice of the most suitable condensation catalyst for a particular application would be readily apparent to those skilled in the art. Preferred catalysts are metal salts of carboxylic acids, for example, lead octoate, stannous acetate or octoate or dibutyl tin dilaurate or diacetate, the most preferred of which is stannous octoate.

The proportion of condensation catalyst, if present, is in the range of 0.25 to 2.5, preferably 0.25 to 1.25, more preferably 0.50 to 1.0, parts by weight of catalyst per 100 parts by weight of vulcanisable polysiloxane.

### Silicone Elastomer - Other Ingredients

In addition to the above-mentioned components of vulcanisable polysiloxane(s), crosslinking agent(s) and, if necessary, condensation catalyst, the silicone elastomer according to the second aspect of the present invention may also contain finely divided fillers or other additives, for example, heat stability additives, dyes, plasticisers, processing aids and pigments. As would be appreciated by those skilled in the art, any of a wide variety of inert organic or inorganic filler materials may be employed including, for example, diatomaceous earth, crushed quartz, calcium carbonate, titania, zirconium silicate and ferric oxide.

The biomimetic silicone elastomers of the second aspect of the invention may be produced by any means known in the art, including extrusion or injection moulding processes. Any suitable polysiloxane known in the art may be used as the starting material. Fillers such as diatomaceous earth may be included to improve mechanical strength. In addition, the crosslinking agent(s) as disclosed in the present invention as described hereinbefore, or a combination of such crosslinking agents may be included with the conventional crosslinking agents (tetrapropoxysilane and tetra-n-butoxysilane). Furthermore, a catalyst known in the art may be included to facilitate the curing reaction, for example, metallic salts of carboxylic acids, of which stannous octoate is particularly preferred. It will be appreciated by those skilled in the art that the amount of catalyst used in the compositions of the invention may be varied in order to obtain the required vulcanisation and/or desired curing time for a given vulcanisable mixture at a given temperature.

Although vulcanisation of such compositions, in the presence of a crosslinking agent can occur at room temperature, it is advantageous to use an elevated temperature in order to speed up the vulcanisation process. Preferably, vulcanisation of the material to produce the novel biomimetic silicone elastomers of the invention is performed at about 80°C, the process typically requiring between 2 and 15 minutes, depending on the crosslinking agent, or agents, chosen for the purpose.

### GENERAL SYNTHETIC METHOD FOR HOMO ALKOXYSILANES

The general reaction scheme for the synthesis of homo alkoxysilanes used as crosslinking agents to produce the novel biomimetic silicone elastomers of the invention is shown in Figure 2, in which R represents an alkyl, alkenyl, alkynyl, aryl or acyl group as already defined.

### Synthesis of tetra(oleyloxy)silane crosslinking agent

Tetrapropoxysilane (10.0 g) was added to oleyl alcohol (40.75 g) in a 100 mL round-bottomed flask. The mixture was refluxed, during which propanol boils off at approx. 97°C. However, both the temperature and reaction time can be decreased if reduced pressure is employed. Yields of tetraoleyloxysilane and propanol were in excess of 95% of the theoretical yield. Product identity was confirmed by proton nuclear magnetic resonance spectroscopy.

Other homo crosslinking agents are prepared in a similar way as described hereinabove, using an organooxysilane and the appropriate alcohol in molar ratios of about 1:4. Other hetero crosslinking agents are prepared in a similar way, using an organo oxysilane and the appropriate alcohols.

### GENERAL METHOD OF MANUFACTURE OF BIOMIMETIC SILICONE ELASTOMERS

A polysiloxane vulcanisable composition is prepared by thoroughly blending a hydroxyl-terminated vulcanisable polysiloxane, or mixture thereof, containing about 25 parts by weight (per 100 parts by weight of the polysiloxane starting material) of diatomaceous earth as the filler, with 1-25, preferably 2-25, more preferably 2-15, most preferably 2-12, parts by weight (per 100 parts by weight of the vulcanisable polysiloxane starting material) of a crosslinking agent or agents as disclosed in the present invention or, alternatively, 1-25, preferably 2-25, more preferably 2-15, most preferably 2-12, parts by weight (per 100 parts by weight of the vulcanisable polysiloxane) of a mixture of a crosslinking agent(s) as disclosed in the present invention with the conventional crosslinking agent (tetrapropoxysilane or tetra-n-butoxysilane). Entrapped air is removed from the mix by an appropriate means, for example, by allowing the mix to stand for several hours. A suitable catalyst, typically stannous octoate (0.25 to 2.5, preferably 0.25 to 1.25, more preferably 0.5 to 1.0 parts by weight per 100 parts by weight of the vulcanisable polysiloxane) is then added rapidly with gentle stirring. Vulcanisation at 80°C for up to 15 minutes (see Table 1) yields the silicone elastomer of the second aspect of the invention. Greater amounts of the catalyst may be added, if a quicker cure time is desired.

A biomimetic silicone elastomer, for medical device applications, may be produced by rapidly processing the catalysed mixture using, for example, single or multiple extrusion, or injection moulding processes. Thus, a range of novel silicone elastomers of the invention were prepared by extruding a mixture of the required amounts of a vulcanisable polysiloxane or a mixture thereof, a crosslinking agent or a mixture thereof and condensation catalyst or a mixture thereof (if present), between two flat glass plates separated by spacers of 2 mm thickness, with curing at 80°C for up to 15 minutes, according to Table 1 (Materials B-I).

Data for a conventional silicone elastomer produced using tetrapropoxysilane, a crosslinking agent known in the art, is included for comparative purposes (Material A). Data for a silicone elastomer produced using tetrapropoxysilane and incorporating an amount of oleyl alcohol equivalent to that produced during the crosslinking reaction of Material F is also included for comparative purposes (Material J). It is of particular significance that Material J failed to cure; even after a 48 hour period, whereas Material F, in which the oleyl alcohol was generated *in situ* by the curing reaction, cured rapidly and Material A, in which the actual crosslinking agent, and its concentration, is identical to that of Material J, also cured very rapidly.

The parts by weight of crosslinking agent used in each case is given per 100 parts by weight of the vulcanisable polysiloxane, together with the curing time required to produce a solid silicone elastomer at room temperature and the parts by weight of condensation catalyst used per 100 parts by weight of the vulcanisable polysiloxane. In each case, the crosslinking agent(s) used for each of Materials A-J is a liquid at room temperature.

**Table 1 :**

| Formulations | | | | | |
|---|---|---|---|---|---|
| | Alcohol starting material for crosslinking agent/by-product | Crosslinking Agent | Parts by weight of crosslinking agent (per 100 parts by weight of the vulcanisable polysiloxane) | Cure time (minutes) | catalyst conc. (parts by weight of stannous octoate per 100 parts by weight of the vulcanisable polysiloxane) |
| A | propan-1-ol | tetra(propyloxy)silane | 2.5 | 2 | 0.50 |
| B | hexan-1-ol | tetra(hexyloxy)silane | 4.0 | 2 | 0.50 |
| C | octan-1-ol | tetra(octyloxy)silane | 5.0 | 3 | 0.50 |
| D | decan-1-ol | tetra(decyloxy)silane | 6.1 | 4 | 0.50 |
| E | oleyl alcohol | tetra(oleyloxy)silane | 10.3 | 2 | 1.0 |
| F | oleyl alcohol | tetra(oleyloxy)silane | 10.3 | 14 | 0.50 |
| G | phenol | tetra(phenoxy)silane | 4 | 2 | 0.50 |
| H | propan-1-ol and | tetra(propyloxy) silane and | 0.50 | 2 | 0.75 |
| | oleyl alcohol | tetra(oleyloxy) silane | 8.30 | | |
| I | propan-1-ol and | tetra(propyloxy) silane and | 0.50 | 5 | 0.50 |
| | oleyl alcohol | tetra(oleyloxy)silane | 8.30 | | |
| J | propan-1-ol | tetra(propyloxy) silane | 2.5 parts +10.2 parts by weight of oleyl alcohol | did not cure after 48 hours | |

### Example 1

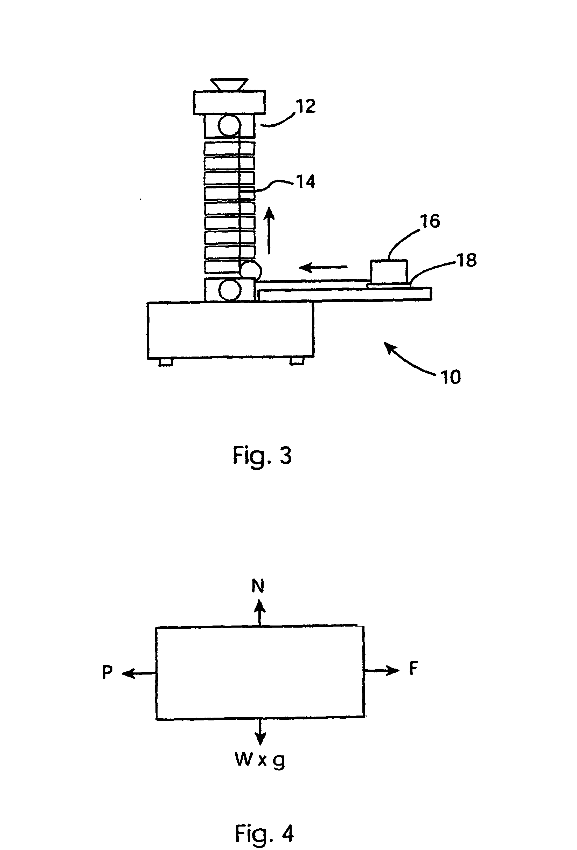

The surface lubricity of novel biomimetic silicone elastomers of the invention prepared according to the general method of manufacture (Material F and Material H, Table 1) were compared to that of the conventional elastomer (Material A, Table 1). Thus, the co-efficient of friction between the respective silicone elastomers and stainless steel was determined using a TA-XT2 Texture Analyser (Stable Micro Systems) customised for this purpose. The experimental set-up is illustrated in Figure 3, in which the apparatus for determining the co-efficient of friction is generally indicated as 10. The apparatus 10 includes an upper clamp attached to a texture analyser crosshead (generally indicated as 12), to which is attached, via a waxed cotton thread 14, a weighted stainless steel sled 16.

At the start of each experiment, the stainless steel sled 16 was placed lightly on the surface of the silicone elastomer 18 attached to the horizontal platform such that the thread 14 attaching it to the upper clamp 12 (via the pulley) was just taut. The crosshead was then moved vertically upwards through a distance of 50mm at a constant speed of 4 mm per second. This caused the sled 16 to move horizontally across the surface of the silicone elastomer 18 towards the pulley through the same distance and at the same speed. The forces required to produce movement were recorded and used to calculate the static and dynamic coefficients of friction as follows;
1. Static friction : calculated from the force required to initiate movement.
2. Dynamic Friction: calculated from the mean force recorded during movement.

The coefficients were calculated by dividing the force required to produce movement by the downward force due to the weight of the sled 16. This can be understood with reference to Figure 4.

Resolving forces horizontally and vertically, we see that;
1. Frictional force opposing motion (F) = Pulling force (P)
2. Reaction force normal to the direction of motion (N) = Downward force due to weight of sled (W), multiplied by acceleration due to gravity (g), i.e., W.g

It is also known that the frictional force equals the normal reaction force multiplied by the co-efficient of friction, m.
i.e. P = F = m.W.g
Therefore ; m=P/(10W)

In all cases, the contact area between the sled and the silicone elastomer was *2.375 x 10*^{*-3*} *m*^{*2*}. For Materials F or E, smooth sliding movement was observed using a sled whose weight (W) was 1.1Kg. However, for comparative Material A, (Table 1), the 1.1 Kg sled did not slide but toppled over under the pulling force.
Therefore, the sled weight was reduced to 0.234Kg for these samples. A summary of the results of friction tests is given in Table 2.

**Table 2:**

| | | |
|---|---|---|
| Static and dynamic co-efficients of friction for silicone elastomers. Each result is the mean ± one standard deviation of eight replicates. | | |

| Material (designation according to Table 1) | Co-efficient of static friction, m ₛ | Co-efficient of dynamic friction, m _{d} |
|---|---|---|
| A | 0.531±0.046 | 0.312 ± 0.042 |
| F | 0.019±0.002 | 0.011±0.002 |
| E | 0.022±0.002 | 0.015±0.002 |

It will be observed that the comparative material (Material A) exhibits considerably higher co-efficients of static friction and of dynamic friction. In marked contrast, silicone elastomers of the present invention exhibit a coefficient of static friction of 0.001 to 0.450, preferably 0.005 to 0.250, more preferably 0.005 to 0.100, most preferably 0.005 to 0.050. Silicone elastomers of the present invention exhibit a co-efficient of dynamic friction of 0.001 to 0.250, preferably 0.005 to 0.100, most preferably 0.005 to 0.050. This means that the silicone elastomers of the present invention have much improved surface lubricity, thereby fulfilling one of the objects of the present invention.

### Example 2

The resistance to encrustation of novel biomimetic silicone elastomers of the invention prepared according to the general method of manufacture (Material F and Material E, Table 1) was compared to that of a standard elastomer (Material A, Table 1). Thus, sections (50 mm x 10 mm x 2 mm) of each material were suspended in an artificial urine model [6]. The artificial urine was changed on a daily basis to simulate conditions in the urinary tract and maintained at 37°C. Five sections of each material were removed after specific times for assessment of encrustation. Encrusted deposits on the respective materials were dissolved in acetic acid and calcium levels (mg cm⁻²) determined by atomic absorption spectrophotometry, the concentration of surface calcium as Ca²⁺ being recognised as the major marker of encrustation in this situation [6]. The data obtained (Table 3) indicates that the silicone elastomers of the present invention (Materials F and E, Table 1) showed an approximately 20 % decrease in encrustation compared to the conventional silicone elastomer (Material A, Table 1) after a 14-day exposure period.

**Table 3**

| | |
|---|---|
| Ca⁺⁺ deposits(mg cm⁻²) removed from each material after 14 days incubation. Each value is the mean of at least three replicates. | |

| Material (designation as per Table 1) | 14 day |
|---|---|
| F | 44.80 |
| E | 45.94 |
| A | 55.34 |

Thus the silicone elastomers of the present invention exhibit Ca⁺⁺ deposits (mg cm⁻²) of 35-52.5, preferably 40-50, most preferably 42.5-47.5.

### Example 3

The surface resistance to adherent microorganisms of novel biomimetic silicone elastomers of the invention prepared according to the general method of manufacture (Material F and Material E, Table 1) was compared to the surface resistance to adherent microorganisms of a standard elastomer prepared with a lower molecular weight crosslinker, yielding n-propanol as the volatile, non-oily by-product (Material A, Table 1). Thus, *Escherichia coli* isolated from microbial biofilm on retrieved urethral stents was grown to stationary phase in Mueller Hinton Broth at 37°C [7]. The bacterial cells were then washed and standardised in sterile phosphate buffered saline (PBS, pH 7.3, 0.01mM) to the required density, expressed as colony forming units (cfu) ml⁻¹. Three discs of each silicone elastomer were contacted with the standardised bacterial culture under shaking conditions (100 rpm, 37°C). The discs were removed at specific times and washed to release non-adherent bacteria. Each disc was then placed in PBS (10ml) and adherent bacteria dislodged by low power sonication and vortexing. The number of adherent bacteria/mm² silicone elastomer was calculated following serial dilution and plating of PBS samples onto Mueller Hinton Agar and incubation at 37°C for 24-48 hours. The data obtained (Table 4) indicates that Materials F and E of the present invention (Table 1) showed an approximately five-fold decrease in the number of adhering microorganisms compared to a standard silicone elastomer (Material A, Table 1) after a 6-hour exposure period.

**Table 4**

| Number of adhering organisms per mm² material. | | |
|---|---|---|
| Material (designation according to Table 1) | 4 hours | 6 hours |
| F | 4.60 x 10³ | 1.05 x 10⁴ |
| E | 4.49 x 10³ | 1.11 x 10⁴ |
| A | 9.47 x 10³ | 5.40 x 10⁴ |

Thus, the silicone elastomers of the present invention exhibit reduced adherence of microorganisms of the order of 2.5-8.5 organisms per mm², preferably 2.5-7.5, most preferably 3.5-5.5 after 4 hours.

### Example 4

The surface hydrophobicity of novel biomimetic silicone elastomers of the invention prepared according to the general method of manufacture (Material F and Material E, Table 1) was compared to that of a standard elastomer (Material A, Table 1) by determining the advancing contact angle of water on each material. Thus, the advancing contact angles for each material were determined using a Cahn Dynamic Contact Analyser with reagent grade 1 water as the wetting medium. Samples (aprox. 10mm x 30mm x 1.6mm) were suspended just above the wetting medium with the longest axis vertical. They were then cyclically lowered and raised seven times through a distance of 10mm at a constant speed of 0.01mm.sec⁻¹. This process was repeated three times for each material. Advancing contact angles were determined from the forces required to lower and raise the material during each cycle. The data obtained (Table 5) indicates that the silicone elastomers of the present invention (Materials F and E, Table 1) showed an approximate decrease of 15 degrees in the advancing contact angle compared to the conventional silicone elastomer (Material A, Table 1). Thus, the silicone elastomers of the present invention exhibit an advancing contact angle in the range of 75°-97.5°, preferably 80°-92.5°, most preferably 85°-90°. This indicates a reduction in surface hydrophobicity and, thus, a reduced propensity to surface attachment of certain microorganisms.

**Table 5**

| | |
|---|---|
| Advancing contact angles of silicone elastomers. Each value is the mean of measurements made during three experiments. | |

| Material (designation according to Table 1) | Advancing Contact Angle (degrees) |
|---|---|
| Material F | 86.16 |
| Material E | 88.29 |
| Material A | 101.22 |

### CITED REFERENCES

1. EP-A- 0 407 868
2. EP-A- 0 407 867
3. M. Alagar and V. Krishnasamy. Preparation and characterisation of tetraalkoxysilanes. Hung. J. Ind. Chem. (1987) 15(4), 453-468 (Chem. Abs. 110: 114897).
4. J. Lukasiak et al. Alkoxysilyl derivatives of methyl, ethyl and propyl-p-hydroxybenzoates. Pamiet. Farm. (1974) 155 (7407), 10 pp. (Chem. Abs. 85: 142765).
5. Falkenberg et al. J. A. Am. Chem Soc. (1947) 69, 487
6. Tunney M.M., Bonner M.C., Keane P.F., Gorman S.P. 1996. Development of a model for assessment of biomaterial encrustation in the upper urinary tract. Biomaterials 17, 1025-1029.
7. Keane P.F., Bonner M.C., Johnston S.R., Zafar A., Gorman S.P. 1994. Characterisation of biofilm and encrustation on stents in vivo. *Br. J. Urol.,* 73, 687-691.

## Claims

1. A polysiloxane vulcanisable composition comprising a vulcanisable polysiloxane or a mixture thereof; a crosslinking agent, or a mixture thereof, the crosslinking agent or the mixture thereof including a silane having four oxygen-bonded substituents, at least one of said oxygen-bonded substituents, which may be the same or different, being selected from functionalised or unfunctionalised, substituted or unsubstituted, straight or branched chain C₅-C₂₅ alkyl; functionalised or unfunctionalised, substituted or unsubstituted C₅₋₂₅ alkenyl or alkynyl; substituted or unsubstituted aryl; or substituted or unsubstituted acyl, in which the moiety bonded to the carboxyl group is a functionalised or unfunctionalised, substituted or unsubstituted, straight or branched chain C₄₋₂₅ alkyl; functionalised or unfunctionalised, substituted or unsubstituted, straight or branched chain C₄₋₂₅ alkenyl or alkynyl or substituted or unsubstituted aryl; and, if necessary, a condensation catalyst.

2. A polysiloxane vulcanisable composition according to Claim 1, in which the amount of the crosslinking agent, or the mixture thereof, lies in the range of 1 to 25 parts, preferably 2 to 25 parts, more preferably 2 to 15 parts, most preferably 2 to 12 parts by weight, per 100 parts by weight of the vulcanisable polysiloxane or the mixture thereof.

3. A polysiloxane vulcanisable composition according to Claim 1 or 2, in which the amount of condensation catalyst lies in the range of 0.25 to 2.5, preferably 0.25 to 1.25, more preferably 0.50 to 1.0, parts by weight of catalyst by 100 parts by weight of the vulcanisable polysiloxane or the mixture thereof.

4. A silicone elastomer formed by vulcanising a vulcanisable polysiloxane, or a mixture thereof, in the presence of a crosslinking agent, or a mixture thereof, the crosslinking agent or the mixture thereof including a silane having four oxygen-bonded substituents, at least one of said oxygen-bonded substituents, which may be the same or different, being selected from functionalised or unfunctionalised, substituted or unsubstituted, straight or branched chain C₅-C₂₅ alkyl; functionalised or unfunctionalised, substituted or unsubstituted, straight or branched chain C₅₋₂₅ alkenyl or alkynyl; substituted or unsubstituted aryl; or substituted or unsubstituted acyl in which the moiety bonded to the carboxyl group is a functionalised or unfunctionalised, substituted or unsubstituted, straight or branched chain C₄₋₂₅ alkyl; functionalised or unfunctionalised, substituted or unsubstituted, straight or branched chain C₄₋₂₅ alkenyl or alkynyl or substituted or unsubstituted aryl; and, if necessary, a condensation catalyst.

5. A medical device comprising a silicone elastomer according to Claim 4.

6. Use of a silicone elastomer according to Claim 4 in a medical device for permanent or temporary implantation in, or for attachment in or on, the human or animal body.

7. Use of a silicone elastomer according to Claim 4 for the manufacture of a medical device for temporary or permanent implantation in, or for attachment in or on, the human or animal body.

8. A silicone elastomer according to Claim 4, in which the amount of the crosslinking agent, or the mixture thereof, lies in the range of 1 to 25 parts, preferably 2 to 25 parts, more preferably 2 to 15 parts, most preferably 2 to 12 parts by weight, per 100 parts by weight of the vulcanisable polysiloxane or the mixture thereof.

9. A silicone elastomer according to Claim 4 or 8, in which the amount of condensation catalyst lies in the range of 0.25 to 2.5, preferably 0.25 to 1.25, more preferably 0.50 to 1.0, parts by weight of catalyst by 100 parts by weight of the vulcanisable polysiloxane or the mixture thereof.

10. A medical device according to Claim 5, in which the medical device is selected from a urinary tract device, preferably a urethral stent or a urinary catheter, an ocular device, preferably a contact lens; an orthopaedic device; a respiratory device; a cardiovascular device; a dental device; a neurological device; a gastrointestinal device; an audiology device; a surgical device, preferably a surgical glove; a footcare device; a wound healing device; or a condom.

11. Use according to Claim 6 or 7, in which the medical device is selected from a urinary tract device, preferably a urethral stent or a urinary catheter; an ocular device, preferably a contact lens; an orthopaedic device; a respiratory device; a cardiovascular device; a dental device; a neurological device; a gastrointestinal device; an audiology device; a surgical device, preferably a surgical glove; a footcare device; a wound healing device; or a condom.

## Patentansprüche

1. Vulkanisierbare Polysiloxan-Zusammensetzung, umfassend ein vulkanisierbares Polysiloxan oder ein Gemisch davon; ein Vernetzungsmittel oder ein Gemisch davon, wobei das Vernetzungsmittel oder das Gemisch davon ein Silan mit vier Sauerstoff-gebundenen Substituenten einschließt, wobei mindestens einer von genannten Sauerstoff-gebundenen Substituenten, die gleich oder unterschiedlich sein können, ausgewählt wird aus funktionalisiertem oder nicht funktionalisiertem, substituiertem oder nicht substituiertem, gerad- oder verzweigkettigem C₅-C₂₅-Alkyl; funktionalisiertem oder nicht funktionalisiertem, substituiertem oder nicht substituiertem C₅₋₂₅-Alkenyl oder -Alkynyl; substituiertem oder nicht substituiertem Aryl; oder substituiertem oder nicht substituiertem Acyl, worin der an die Carboxylgruppe gebundene Anteil ein funktionalisiertes oder nicht funktionalisiertes, substituiertes oder nicht substituiertes, gerad- oder verzweigtkettiges C₄₋₂₅-Alkyl; funktionalisiertes oder nicht funktionalisiertes, substituiertes oder nicht substituiertes, gerad- oder verzweigtkettiges C₄₋₂₅-Alkenyl- oder -Alkynyl oder substituiertes oder nicht substituiertes Aryl; und falls notwendig, ein Kondensationskatalysator ist.

2. Vulkanisierbare Polysiloxan-Zusammensetzung nach Anspruch 1, worin die Menge des Vernetzungsmittels oder des Gemisches davon in dem Bereich von 1 bis 25 Gewichtsteilen, bevorzugt 2 bis 25 Gewichtsteilen, bevorzugter 2 bis 15 Gewichtsteilen, am bevorzugtesten 2 bis 12 Gewichtsteilen pro 100 Gewichtsteile des vulkanisierbaren Polysiloxans oder des Gemisches davon liegt.

3. Vulkanisierbare Polysiloxan-Zusammensetzung nach Anspruch 1 oder 2, worin die Kondensationskatalysatormenge in dem Bereich von 0,25 bis 2,5, bevorzugt 0,25 bis 1,25, bevorzugter 0,50 bis 1,0 Gewichtsteilen Katalysator pro 100 Gewichtsteile des vulkanisierbaren Polysiloxans oder des Gemisches davon liegt.

4. Silikonelastomer, gebildet durch Vulkanisieren eines vulkanisierbaren Polysiloxans oder eines Gemisches davon, bei Vorliegen eines Vernetzungsmittels oder eines Gemisches davon, wobei das Vernetzungsmittel oder das Gemisch davon ein Silan mit vier Sauerstoff-gebundenen Substituenten einschließt, wobei mindestens einer von genannten Sauerstoff-gebundenen Substituenten, die gleich oder unterschiedlich sein können, ausgewählt wird aus funktionalisiertem oder nicht funktionalisiertem, substituiertem oder nicht substituiertem, gerad- oder verzweigtkettigem C₅-C₂₅-Alkyl; funktionalisiertem oder nicht funktionalisiertem, substituiertem oder nicht substituiertem, gerad- oder verzweigtkettigem C₅₋₂₅-Alkenyl oder -Alkynyl; substituiertem oder nicht substituiertem Aryl; oder substituiertem oder nicht substituiertem Acyl, worin der an die Carboxylgruppe gebundene Anteil ein funktionalisiertes oder nicht funktionalisiertes, substituiertes oder nicht substituiertes, gerad- oder verzweigtkettiges C₄₋₂₅-Alkyl; funktionalisiertes oder nicht funktionalisiertes, substituiertes oder nicht substituiertes, gerad- oder verzweigtkettiges C₄₋₂₅-Alkenyl oder -Alkynyl oder substituiertes oder nicht substituiertes Aryl; und, falls notwendig, ein Kondensationskatalysator ist.

5. Medizinvorrichtung, umfassend ein Silikonelastomer nach Anspruch 4.

6. Verwendung eines Silikonelastomers nach Anspruch 4 in einer Medizinvorrichtung für die permanente oder temporäre Implantation in oder zum Befestigen in oder an dem menschlichen oder tierischen Körper.

7. Verwendung eines Silikonelastomers nach Anspruch 4 für die Herstellung einer Medizinvorrichtung für die temporäre oder permanente Implantation in oder zum Befestigen in oder an dem menschlichen oder tierischen Körper.

8. Silikonelastomer nach Anspruch 4, worin die Menge des Vernetzungsmittels oder des Gemisches davon in dem Bereich von 1 bis 25 Gewichtsteilen, bevorzugt 2 bis 25 Gewichtsteilen, bevorzugter 2 bis 15 Gewichtsteilen, am bevorzugtesten 2 bis 12 Gewichtsteilen pro 100 Gewichtsteile des vulkanisierbaren Polysiloxans oder des Gemisches davon liegt.

9. Silikonelastomer nach Anspruch 4 oder 8, worin die Kondensationskatalysatormenge in dem Bereich von 0,25 bis 2,5, bevorzugt 0,25 bis 1,25, bevorzugter 0,50 bis 1,0 Gewichtsteilen Katalysator pro 100 Gewichtsteile des vulkanisierbaren Polysiloxans oder des Gemisches davon enthält.

10. Medizinvorrichtung nach Anspruch 5, worin die Medizinvorrichtung ausgewählt wird aus einer Harnwegsvorrichtung, bevorzugt einem urethralen Stent oder einem Harnblasenkatheter; einer okulären Vorrichtung, bevorzugt einer Kontaktlinse; einer orthopädischen Vorrichtung; einer Atmungsvorrichtung; einer kardiovaskulären Vorrichtung; einer Dentalvorrichtung; einer neurologischen Vorrichtung; einer gastrointestinalen Vorrichtung, einer Audiologie-Vorrichtung; einer chirurgischen Vorrichtung; bevorzugt einem chirurgischen Handschuh; einer Fußpflegevorrichtung; einer Wundheilungsvorrichtung oder einem Kondom.

11. Verwendung nach Anspruch 6 oder 7, worin die Medizinvorrichtung ausgewählt wird aus einer Harnwegsvorrichtung, bevorzugt einem urethralen Stent oder einem Harnblasenkatheter, einer okulären Vorrichtung; bevorzugt einer Kontaktlinse; einer orthopädischen Vorrichtung; einer Atmungsvorrichtung; einer kardiovaskulären Vorrichtung; einer Dentalvorrichtung; einer neurologischen Vorrichtung; einer gastrointestinalen Vorrichtung; einer Audiologie-Vorrichtung; einer chirurgischen Vorrichtung, bevorzugt einem chirurgischen Handschuh; einer Fußpflegevorrichtung; einer Wundheilungsvorrichtung; oder einem Kondom.

## Revendications

1. Composition vulcanisable au polysiloxane, comprenant un polysiloxane vulcanisable ou un mélange de celui-ci ; un agent de réticulation, ou un mélange de celui-ci, l'agent de réticulation ou le mélange de celui-ci comportant un silane ayant quatre substituants liés à l'oxygène, au moins l'un desdits substituants liés à l'oxygène, qui peuvent être identiques ou différents, étant choisi parmi alkyle en C₅-C₂₅ à chaîne linéaire ou ramifiée, substitué ou non substitué, fonctionnalisé ou non fonctionnalisé ; alkényle ou alkynyle en C₅₋₂₅ substitué ou non substitué, fonctionnalisé ou non fonctionnalisé ; aryle substitué ou non substitué ; ou acyle substitué ou non substitué, dans lequel le motif lié au groupement carboxyle est alkyle en C₄₋₂₅ à chaîne linéaire ou ramifiée, substitué ou non substitué, fonctionnalisé ou non fonctionnalisé ; alkényle ou alkynyle en C₄₋₂₅ à chaîne linéaire ou ramifiée, substitué ou non substitué, fonctionnalisé ou non fonctionnalisé, ou aryle substitué ou non substitué ; et, le cas échéant, un catalyseur de condensation.

2. Composition vulcanisable au polysiloxane selon la revendication 1, dans laquelle la quantité de l'agent de réticulation, ou du mélange de celui-ci, se trouve dans la plage allant de 1 à 25 parties, préférablement de 2 à 25 parties, plus préférablement de 2 à 15 parties, tout préférablement de 2 à 12 parties en poids, pour 100 parties en poids du polysiloxane vulcanisable ou du mélange de celui-ci.

3. Composition vulcanisable au polysiloxane selon la revendication 1 ou 2, dans laquelle la quantité de catalyseur de condensation se trouve dans la plage allant de 0,25 à 2,5, préférablement de 0,25 à 1,25, plus préférablement de 0,50 à 1,0 partie en poids de catalyseur pour 100 parties en poids du polysiloxane vulcanisable ou du mélange de celui-ci.

4. Elastomère siliconé formé par la vulcanisation d'un polysiloxane vulcanisable, ou d'un mélange de celui-ci, en présence d'un agent de réticulation, ou d'un mélange de celui-ci, l'agent de réticulation ou le mélange de celui-ci comportant un silane ayant quatre substituants liés à l'oxygène, au moins l'un desdits substituants liés à l'oxygène, qui peuvent être identiques ou différents, étant choisi parmi alkyle en C₅-C₂₅ à chaîne linéaire ou ramifiée, substitué ou non substitué, fonctionnalisé ou non fonctionnalisé ; alkényle ou alkynyle en C₅₋₂₅ à chaîne linéaire ou ramifiée, substitué ou non substitué, fonctionnalisé ou non fonctionnalisé ; aryle substitué ou non substitué ; ou acyle substitué ou non substitué, dans lequel le motif lié au groupement carboxyle est alkyle en C₄-₂₅ à chaîne linéaire ou ramifiée, substitué ou non substitué, fonctionnalisé ou non fonctionnalisé ; alkényle ou alkynyle en C₄-₂₅ à chaîne linéaire ou ramifiée, substitué ou non substitué, fonctionnalisé ou non fonctionnalisé, ou aryle substitué ou non substitué ; et, le cas échéant, un catalyseur de condensation.

5. Dispositif médical comprenant un élastomère siliconé selon la revendication 4.

6. Utilisation d'un élastomère siliconé selon la revendication 4 dans un dispositif médical pour une implantation temporaire ou permanente dans, ou pour une fixation dans ou sur, le corps humain ou animal.

7. Utilisation d'un élastomère siliconé selon la revendication 4 pour la fabrication d'un dispositif médical pour une implantation temporaire ou permanente dans, ou pour une fixation dans ou sur, le corps humain ou animal.

8. Elastomère siliconé selon la revendication 4, dans lequel la quantité de l'agent de réticulation, ou du mélange de celui-ci, se trouve dans la plage allant de 1 à 25 parties, préférablement de 2 à 25 parties, plus préférablement de 2 à 15 parties, tout préférablement de 2 à 12 parties en poids, pour 100 parties en poids du polysiloxane vulcanisable ou du mélange de celui-ci.

9. Elastomère siliconé selon la revendication 4 ou 8, dans lequel la quantité de catalyseur de condensation se trouve dans la plage allant de 0,25 à 2,5, préférablement de 0,25 à 1,25, plus préférablement de 0,50 à 1,0 partie en poids de catalyseur pour 100 parties en poids du polysiloxane vulcanisable ou du mélange de celui-ci.

10. Dispositif médical selon la revendication 5, dans lequel le dispositif médical est choisi parmi un dispositif du tractus urinaire, préférablement un stent urétral ou un cathéter urinaire ; un dispositif oculaire, préférablement une lentille de contact ; un dispositif orthopédique ; un dispositif respiratoire ; un dispositif cardiovasculaire ; un dispositif dentaire ; un dispositif neurologique ; un dispositif gastro-intestinal ; un dispositif d'audiologie ; un dispositif chirurgical, préférablement un gant chirurgical ; un dispositif de pédicure ; un dispositif de cicatrisation de plaies ; ou un préservatif.

11. Utilisation selon la revendication 6 ou 7, dans laquelle le dispositif médical est choisi parmi un dispositif du tractus urinaire, préférablement un stent urétral ou un cathéter urinaire ; un dispositif oculaire, préférablement une lentille de contact ; un dispositif orthopédique ; un dispositif respiratoire ; un dispositif cardiovasculaire ; un dispositif dentaire ; un dispositif neurologique ; un dispositif gastro-intestinal ; un dispositif d'audiologie ; un dispositif chirurgical, préférablement un gant chirurgical ; un dispositif de pédicure ; un dispositif de cicatrisation de plaies ; ou un préservatif.
